# EUROPEAN PATENT SPECIFICATION

(11) **EP 4 042 960 B1**
(45) Date of publication and mention of the grant of the patent: **06.03.2024**
(21) Application number: 20873187.7
(22) Date of filing: 29.09.2020
(51) Int. Cl.: A61B 18/14

(54) **MEDICAL DEVICE**
MEDIZINISCHE VORRICHTUNG
DISPOSITIF MÉDICAL

(30) Priority: 30.09.2019 JP 2019178330
(43) Date of publication of application: 17.08.2022
(73) Proprietor: TERUMO Kabushiki Kaisha, Tokyo 151-0072 (JP)
(72) Inventor: TAKAHASHI, Yusuke, Ashigarakami-gun, Kanagawa 259-0151 (JP)
(74) Representative: Casalonga
(86) International application number: PCT/JP2020/036817
(87) International publication number: WO 2021/065875

(56) References cited:
- WO-A1-2019/009254
- WO-A1-2019/009254
- JP-A- 2009 500 052
- JP-A- 2017 113 538
- US-A1- 2011 106 074
- US-A1- 2017 143 227
- US-A1- 2017 156 790
- US-A1- 2018 271 595

## Description

### Technical Field

The present invention relates to a medical device that expands and maintains a hole of a biological tissue.

### Background Art

Chronic heart failure is one of known heart diseases. The chronic heart failure is broadly classified into a systolic heart failure and a diastolic heart failure, based on a cardiac function index. In a patient suffering from the diastolic heart failure, a myocardium is hypertrophied and increases in stiffness (hardness), so that the blood pressure in a left atrium increases and the pumping function of a heart is decreased. Accordingly, the patient shows a heart failure symptom such as a pulmonary edema. There is also a heart disease in which the blood pressure on a right atrium side increases due to pulmonary hypertension or the like, and the pump function of a heart is decreased, thereby showing heart failure symptoms.

In recent years, for the patients suffering from a heart failure, attention has been paid to a shunt treatment in which a shunt (through-hole) serving as an escape route for increased atrial pressure is formed in an atrial septum, thereby being able to reduce heart failure symptoms. In the shunt treatment, the atrial septum is accessed using a transvenous approach method, and a through-hole of a desired size is formed. Then, a method has been known in which a shunt hole is subjected to energy to be cauterized, thereby maintaining the shunt hole.

In addition, for example, PTL 1 discloses a device that brings an expansion body including a plurality of wires having a cauterization function, into contact with a renal artery wall and cauterizes renal sympathetic nerves adjacent to the renal artery wall, as a treatment for hypertension.

### Citation List

### Patent Literature

PTL 1: Japanese Patent No. 6013186

A medical device according to the preamble of claim 1 is known from WO 2019/009254 A1.

### Summary of Invention

### Technical Problem

When the expansion body including the plurality of wires is contracted, the position of the expansion body with respect to a shaft located at a center of the plurality of wires may be displaced. In a case where the positions of the contracted wires are displaced with respect to the shaft, when the expansion body is stored in a tubular member, the expansion body may be damaged or may be difficult to store in the tubular member.

The invention is conceived in view of the aforementioned problems, and an object of the invention is to provide a medical device capable of suppressing damage to an expansion body that expands a biological tissue, or suppressing the difficulty in storing the expansion body in a tubular member when the expansion body is stored in the tubular member.

### Solution to Problem

According to one aspect of the invention to achieve the object, there is provided a medical device including: a shaft portion that is elongate; and an expansion body provided at a distal portion of the shaft portion to be expandable and contractable in a radial direction. The shaft portion includes a central shaft extending along an axis of the expansion body. The expansion body includes a plurality of wire portions that are expandable and contractable in the radial direction. Each of the wire portions includes a contact portion that comes into contact with the central shaft, when contracted. An opening portion is formed in at least one of the contact portion and the central shaft. A protruding portion is formed in at least one of the contact portion and the central shaft. When the expansion body is contracted, at least a part of the protruding portion enters an inside of the opening portion, and comes into contact with the opening portion.

### Advantageous Effect of Invention

In the medical device configured as described above, when the expansion body is stored in a tubular member, it is possible to suppress the displacement of the contact portion of the expansion body to contract, from the central shaft. For this reason, it is possible to suppress damage to the expansion body or to suppress the difficulty in storing the expansion body in the tubular member when the expansion body is stored in the tubular member.

The opening portion and/or the protruding portion may extend in an axial direction. Accordingly, even when the axial positions of the contact portion of the expansion body to contract and of the central shaft are changed, it is possible to suppress the displacement of the contact portion from the central shaft. Note that in a case where one of the opening portion and the protruding portion extends in the axial direction, even when the other does not extend in the axial direction, the opening portion and the protruding portion are slidable on each other without being displaced from each other. Note that both the opening portion and the protruding portion may extend in the axial direction.

When the expansion body is contracted, the contact portion or the central shaft may come into contact with the opening portion so as to be slidable in an axial direction. Accordingly, in the medical device, when the expansion body is stored in the tubular member, the contact portion of the expansion body to contract is slidable in the axial direction without being displaced from the central shaft. For this reason, it is possible to suppress damage to the expansion body or to suppress the difficulty in storing the expansion body in the tubular body when the expansion body is stored in the tubular member.

The wire portion may include a proximal side outward projection portion protruding outward in the radial direction, a distal side outward projection portion located closer to a distal side than the proximal side outward projection portion, to protrude outward in the radial direction, and an inward projection portion protruding inward in the radial direction between the proximal side outward projection portion and the distal side outward projection portion. The contact portion may be formed in the inward projection portion. Accordingly, when the proximal side outward projection portion is contracted and stored in the tubular member from a proximal side, the contact portion of the inward projection portion can come into contact with the central shaft. At this time, since the protruding portion can enter and come into contact with the opening portion, when the proximal side outward projection portion is stored in the tubular member, it is possible to suppress the displacement of the contact portion from the central shaft.

The medical device may further include an energy transmission element disposed on the wire portion to output energy. Accordingly, it is possible to suppress the displacement of the contact portion of the wire portion on which the energy transmission element is disposed, from the central shaft.

The opening portion may be a through-hole. Accordingly, the structure of the medical device can be simplified to reduce the diameter and to reduce the cost.

The opening portion may be a non-through recessed portion. Accordingly, the opening portion does not penetrate through the contact portion or through the central shaft in which the opening portion is provided, to an opposite side. For this reason, it is possible to suppress interference of the opening portion with other members.

An axially orthogonal cross section of an outer peripheral surface of the central shaft may be a substantially circular shape, and the protruding portion may be a part of the outer peripheral surface of the central shaft. Accordingly, the outer peripheral surface of the central shaft can serve as the protruding portion, so that the structure of the medical device can be simplified to reduce the diameter and to reduce the cost. In addition, since the protruding portion is smooth due to the outer peripheral surface of the central shaft serving as the protruding portion, it is possible to suppress interference of the protruding portion with other members.

The protruding portion may be a member protruding outward in the radial direction from an outer peripheral surface of the central shaft.
Accordingly, the protruding portion protruding from the outer peripheral surface of the central shaft can reliably enter the inside of the opening portion of the contact portion, and come into contact with the opening portion. For this reason, the protruding portion can effectively suppress the displacement of the protruding portion that has entered the inside of the opening portion, from the opening portion.

The opening portion may be formed of two opening side protruding portions arranged at an interval. Accordingly, the opening side protruding portions can effectively suppress the displacement of the protruding portion that has entered the inside of the opening portion, from the opening portion 59.

### Brief Description of Drawings

[Fig. 1] Fig. 1 is a front view illustrating an overall configuration of a medical device according to the present embodiment.
[Fig. 2] Fig. 2 is an enlarged perspective view of the vicinity of an expansion body.
[Fig. 3] Fig. 3 is a front view illustrating a state where one wire portion is flattened.
[Fig. 4] Fig. 4 is a view for describing a treatment method, not under the scope of the present invention, using the medical device according to the present embodiment, and is a view for schematically describing a state where the expansion body is disposed in a through-hole of an atrial septum, in which the medical device and a biological tissue are illustrated in a front view and in a cross-sectional view, respectively.
[Fig. 5] Fig. 5 is a view for schematically describing a state where the expansion body is disposed in the atrial septum, in which the medical device and the biological tissue are illustrated in a front view and in a cross-sectional view, respectively.
[Fig. 6] Fig. 6 is a view for schematically describing a state where the expansion body is expanded in the atrial septum, in which the medical device and the biological tissue are illustrated in a front view and in a cross-sectional view, respectively.
[Fig. 7] Fig. 7 is a cross-sectional view illustrating a state where the expansion body is stored in a storage sheath, Fig. 7(A) illustrates a state where a proximal portion of the expansion body is stored, and Fig. 7(B) illustrates a state where the entirety of the expansion body is stored.
[Fig. 8] Fig. 8 is a cross-sectional view taken along line A-A in Fig. 7(A).
[Fig. 9] Fig. 9 is a perspective view illustrating a state where the proximal portion of the expansion body is stored in the storage sheath.
[Fig. 10] Fig. 10 illustrates cross-sectional views of the vicinities of contact portions of medical devices according to modification examples, Fig. 10(A) illustrates a first modification example, Fig. 10(B) illustrates a second modification example, Fig. 10(C) illustrates a third modification example, and Fig. 10(D) illustrates a fourth modification example.

### Description of Embodiments

Hereinafter, embodiments of the invention will be described with reference to the accompanying drawings. Note that dimensional ratios in the drawings may be exaggerated and different from actual ratios for convenience of description. In addition, in the specification, a side on which a medical device 10 is inserted into a biological lumen will be referred to as a "distal side", and a side on which operation is performed will be referred to as a "proximal side".

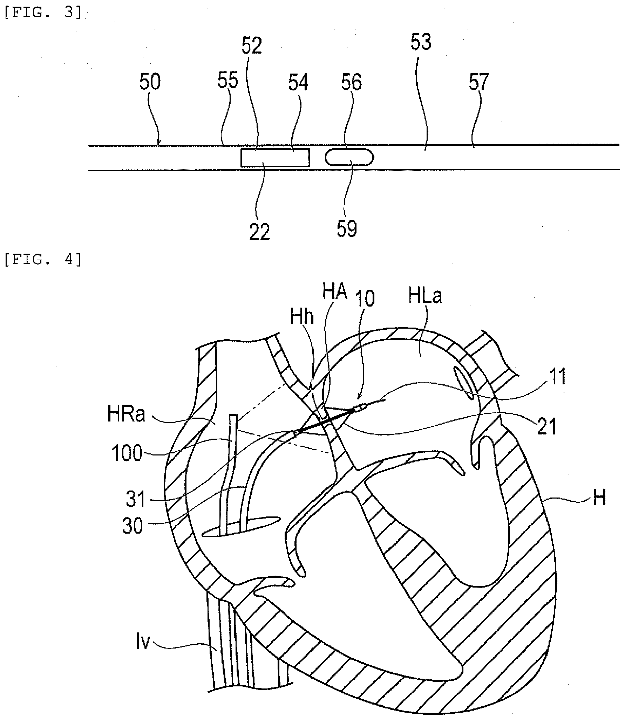

As illustrated in Fig. 4, the medical device 10 according to the present embodiment is configured to be able to expand a through-hole Hh formed in an atrial septum HA of a heart H of a patient and to perform a maintenance treatment to maintain the size of the expanded through-hole Hh.

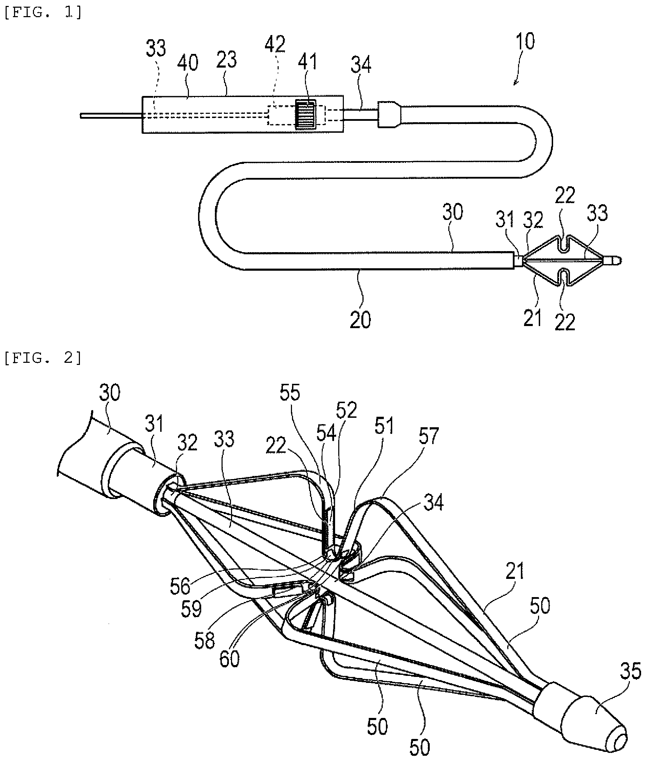

As illustrated in FIG. 1, the medical device 10 according to the present embodiment includes a shaft portion 20 that is elongate, an expansion body 21 provided at a distal portion of the shaft portion 20, and an operation unit 23 provided at a proximal portion of the shaft portion 20. The expansion body 21 is provided with an energy transmission element 22 for performing the aforementioned maintenance treatment.

The shaft portion 20 includes an outer shaft 31 that holds the expansion body 21 at the distal portion of the outer shaft 31, and a storage sheath 30 that stores the outer shaft 31. The storage sheath 30 is movable forward and backward with respect to the outer shaft 31 in an axial direction. In a state where the storage sheath 30 is moved to a distal side of the shaft portion 20, the storage sheath 30 can store the expansion body 21 thereinside. The storage sheath 30 is moved to the proximal side from a state where the expansion body 21 is stored, and thus the expansion body 21 can be exposed.

A central shaft 33 is stored inside the outer shaft 31. The central shaft 33 is a shaft for pulling to cause a compression force to act on the expansion body 21. An axially orthogonal cross section of an outer peripheral surface of the central shaft 33 is a substantially circular shape. The central shaft 33 protrudes from a distal end of the outer shaft 31 to the distal side, and a distal portion of the central shaft 33 is fixed to a distal member 35. A proximal portion of the central shaft 33 is led out to the proximal side from the operation unit 23. The distal member 35 to which the distal portion of the central shaft 33 is fixed may not be fixed to the expansion body 21. Accordingly, the distal member 35 can pull the expansion body 21 in a compression direction. In addition, when the expansion body 21 is stored in the storage sheath 30, the distal member 35 is separated to the distal side from the expansion body 21, so that the expansion body 21 can be easily moved in a stretching direction and storability can be improved.

The operation unit 23 includes a housing 40 to be gripped by an operator, an operation dial 41 to be rotationally operable by the operator, and a conversion mechanism 42 that operates in conjunction with rotation of the operation dial 41. The central shaft 33 is held by the conversion mechanism 42 inside the operation unit 23. The conversion mechanism 42 can move the held central shaft 33 forward and backward along the axial direction with rotation of the operation dial 41. For example, a rack and pinion mechanism can be used as the conversion mechanism 42.

The expansion body 21 will be described in more detail. As illustrated in Figs. 2 and 3, the expansion body 21 includes a plurality of wire portions 50 in a circumferential direction. In the present embodiment, four wire portions 50 are provided in the circumferential direction. Note that the number of the wire portions 50 is not particularly limited. Each of the wire portions 50 is expandable and contractable in a radial direction of the expansion body 21. In a natural state where no external force acts on the expansion body 21, the expansion body 21 is in a reference form where the expansion body 21 is deployed in the radial direction. A proximal portion of the wire portion 50 extends from a distal portion of the outer shaft 31 to the distal side. A distal portion of the wire portion 50 extends from a proximal portion of the distal member 35 to the proximal side. The wire portion 50 is inclined such that the size in the radial direction increases from both end portions toward a central portion in the axial direction. In addition, the wire portion 50 includes a holding portion 51 having a valley shape in the radial direction of the expansion body 21, at the central portion of the wire portion 50 in the axial direction.

The holding portion 51 includes a proximal side holding portion 52, and a distal side holding portion 53 located closer to the distal side than the proximal side holding portion 52. The holding portion 51 further includes a proximal side outward projection portion 55, an inward projection portion 56, and a distal side outward projection portion 57. It is preferable that in the reference form, an interval between the proximal side holding portion 52 and the distal side holding portion 53 in the axial direction is slightly wider on a radially outward side than on a radially inward side. Accordingly, a biological tissue is easily disposed between the proximal side holding portion 52 and the distal side holding portion 53 from the radially outward side.

The proximal side holding portion 52 includes a projection portion 54 protruding toward the distal side. The energy transmission element 22 is disposed on the projection portion 54. Note that the proximal side holding portion 52 may not include the projection portion 54. Namely, the energy transmission element 22 may not protrude to the distal side.

The proximal side outward projection portion 55 is located on a proximal side of the proximal side holding portion 52, and is formed in a shape projecting outward in the radial direction. The proximal side outward projection portion 55 is stored in the storage sheath 30, thus being deformable from a projection shape into a shape close to being flat.

The distal side outward projection portion 57 is located on a distal side of the distal side holding portion 53, and is formed in a shape projecting outward in the radial direction. The proximal side outward projection portion 55 is stored in the storage sheath 30, thus being deformable from a projection shape into a shape close to being flat.

The inward projection portion 56 is located between the proximal side holding portion 52 and the distal side holding portion 53, and is formed in a shape projecting inward in the radial direction. A contact portion 58 that can come into contact with the central shaft 33 is formed on a radially inner side of the inward projection portion 56. The contact portion 58 includes an opening portion 59 that can face the central shaft 33, and two outer edge portions 60 interposing the opening portion 59 in a width direction. The width direction is a direction orthogonal to the axial direction of the expansion body 21, and is a direction orthogonal to the radial direction of the expansion body 21. In the present embodiment, the opening portion 59 is a through-hole penetrating through the inward projection portion 56 in the radial direction of the expansion body 21. Note that the opening portion 59 may be formed not only in the inward projection portion 56 but also to the proximal side holding portion 52 or to the distal side holding portion 53. The inward projection portion 56 in which the opening portion 59 is provided has low flexural rigidity. For this reason, the inward projection portion 56 is easily deformed into a shape projecting inward in the radial direction, and is easily deformed into a shape close to being flat.

In the present embodiment, the energy transmission element 22 is provided at the proximal side holding portion 52, but the energy transmission element 22 may be provided at the distal side holding portion 53.

Each of the wire portions 50 forming the expansion body 21 has, for example, a flat plate shape obtained by cutting a cylinder. A wire forming the expansion body 21 can have a thickness of 50 to 500 µm and a width of 0.3 to 2.0 mm. However, a wire forming the expansion body 21 may have dimensions outside these ranges. In addition, the shape of the wire portion 50 is not limited, and may have, for example, a circular cross-sectional shape or other cross-sectional shapes.

Since the energy transmission element 22 is provided at the projection portion 54 of the proximal side holding portion 52, when the holding portion 51 holds the atrial septum HA, energy from the energy transmission element 22 is transmitted from a right atrium side to the atrial septum HA. Note that when the energy transmission element 22 is provided at the distal side holding portion 53, energy from the energy transmission element 22 is transmitted from a left atrium side to the atrial septum HA.

The energy transmission element 22 is configured as, for example, a bipolar electrode that receives electric energy from an energy supply device (not illustrated) that is an external device. In this case, energization is performed between the energy transmission elements 22 disposed on the wire portions 50. The energy transmission element 22 and the energy supply device are connected to each other by a conducting wire (not illustrated) coated with an insulating coating material. The conducting wire is led out to the outside via the shaft portion 20 and via the operation unit 23, and is connected to the energy supply device.

Alternatively, the energy transmission element 22 may be configured as a monopolar electrode. In this case, energization is performed between the energy transmission element 22 and a counter electrode plate prepared outside a body. In addition, the energy transmission element 22 may be a heating element (electrode chip) that receives high-frequency electric energy from the energy supply device to generate heat. In this case, energization is performed between the energy transmission elements 22 disposed on the wire portions 50. Further, the energy transmission element 22 can be configured as an element capable of applying energy to the through-hole Hh, such as an element that exerts a heating or cooling action using microwave energy, ultrasound energy, coherent light such as laser, a heated fluid, a cooled fluid, or a chemical medium, an element that generates frictional heat, or a heater including an electric wire or the like, and a specific form of the energy transmission element 22 is not particularly limited.

The wire portion 50 can be made of a metallic material. As the metallic material, for example, a titanium-based (Ti-Ni, Ti-Pd, Ti-Nb-Sn, or the like) alloy, a copper-based alloy, stainless steel, β-titanium steel, or a Co-Cr alloy can be used. Note that it is better to use an alloy or the like having a spring property such as a nickel-titanium alloy. However, the material for the wire portion 50 is not limited to these materials, and the wire portion 50 may be made of other materials.

The shaft portion 20 includes an inner shaft 32 inside the outer shaft 31, and the central shaft 33 is stored inside the inner shaft 32. A guide wire lumen is formed in the central shaft 33 and in the distal member 35 along the axial direction, and a guide wire 11 can be inserted into the guide wire lumen.

It is preferable that the storage sheath 30, the outer shaft 31, and the inner shaft 32 of the shaft portion 20 are made of a material having a certain degree of flexibility. Examples of such a material include polyolefins such as polyethylene, polypropylene, polybutene, ethylene-propylene copolymer, ethylene-vinyl acetate copolymer, ionomer, and a mixture of two or more thereof, fluororesins such as soft polyvinyl chloride resin, polyamide, polyamide elastomer, polyether blockamide, polyester, polyester elastomer, polyurethane, and polytetrafluoroethylene, polyimide, PEEK, silicone rubber, and latex rubber.

The central shaft 33 can be made of, for example, an elongate wire such as a metallic material such as stainless steel or a super-elastic alloy such as a nickel-titanium alloy or a copper-zinc alloy, or a resin material having relatively high rigidity. In addition, the central shaft 33 may be formed by coating the above material with a resin material such as polyvinyl chloride, polyethylene, polypropylene, ethylene-propylene copolymer, or fluororesin.

The distal member 35 can be made of, for example, a metallic material such as stainless steel or a super-elastic alloy such as a nickel-titanium alloy or a copper-zinc alloy or a resin material having relatively high rigidity.

Next, a treatment method, not under the scope of the present invention, using the medical device 10 according to the present embodiment will be described. The treatment method is performed on a patient suffering from a heart failure (left heart failure). More specifically, as illustrated in Fig. 4, the treatment method is performed on a patient suffering from a chronic heart failure in which a myocardium of a left ventricle of the heart H is hypertrophied and increases in stiffness (hardness) to cause an increase in blood pressure in a left atrium HLa.

When the operator forms the through-hole Hh, the operator delivers an introducer in which a guiding sheath and a dilator are combined together, to the vicinity of the atrial septum HA. The introducer can be delivered to, for example, a right atrium HRa via an inferior vena cava Iv. In addition, the introducer can be delivered using the guide wire 11. The operator can insert the guide wire 11 into the dilator, and deliver the introducer along the guide wire 11. Note that the insertion of the introducer into or the insertion of the guide wire 11 into a living body can be performed using a known method such as using an introducer for blood vessel introduction.

Next, the operator causes a puncture device (not illustrated) and the dilator to penetrate through the atrial septum HA from a right atrium HRa side toward a left atrium HLa side to form the through-hole Hh. For example, a device such as a wire having a sharp distal end can be used as the puncture device. The puncture device is inserted into the dilator, and is delivered to the atrial septum HA. After the guide wire 11 is removed from the dilator, instead of the guide wire 11, the puncture device can be delivered to the atrial septum HA.

Next, the medical device 10 is delivered to the vicinity of the atrial septum HA along the guide wire 11 inserted into the left atrium HLa from the right atrium HRa via the through-hole Hh in advance. At this time, a part of a distal portion of the medical device 10 passes through the through-hole Hh opened in the atrial septum HA, and reaches the left atrium HLa. When the medical device 10 is inserted, the expansion body 21 is in a contracted form where the expansion body 21 is stored in the storage sheath 30. In the contracted form, the proximal side outward projection portion 55, the inward projection portion 56, and the distal side outward projection portion 57 that have a projection shape in a natural state are deformed into a shape close to being flat, and thus the expansion body 21 is contracted in the radial direction.

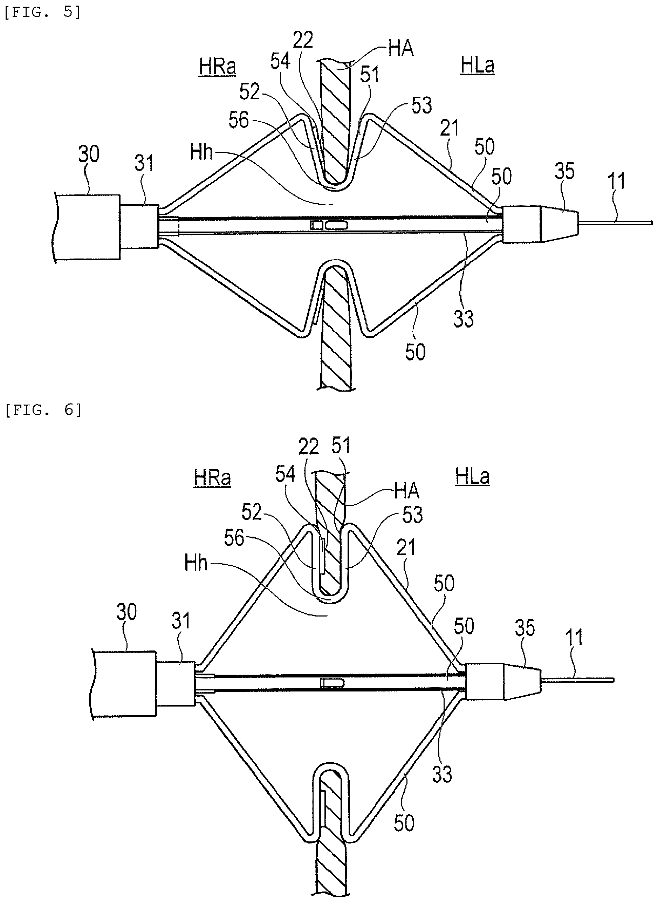

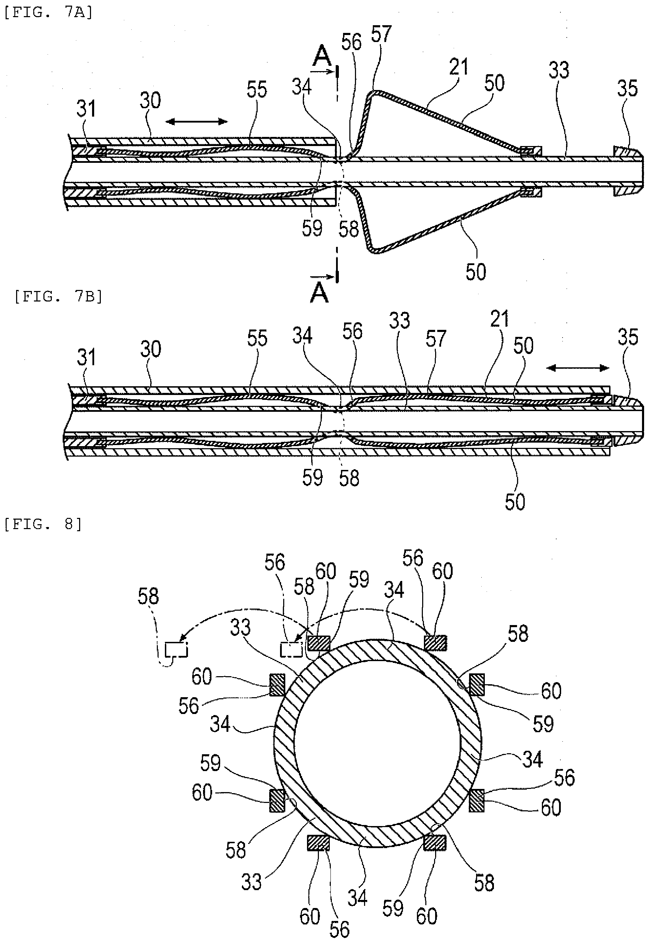

Next, as illustrated in Fig. 7(A), the storage sheath 30 is moved to the proximal side to expose a distal side portion of the expansion body 21 into the left atrium HLa. Accordingly, the distal side portion of the expansion body 21 is deployed in the radial direction inside the left atrium HLa by its own restoring force. Next, as illustrated in Fig. 5, the storage sheath 30 is moved to the proximal side to expose the entirety of the expansion body 21. Accordingly, a proximal side portion of the expansion body 21 is deployed in the radial direction inside the right atrium HRa by its own restoring force. At this time, the inward projection portion 56 is disposed inside the through-hole Hh. Accordingly, the entirety of the expansion body 21 is deployed by its own restoring force, and restores to the original reference form or to a form close to the reference form. At this time, the atrial septum HA is disposed between the proximal side holding portion 52 and the distal side holding portion 53. Note that the expansion body 21 may come into contact with the through-hole Hh, thereby returning to a shape close to the reference form instead of completely returning to the reference form. Note that in this state, the expansion body 21 is not covered with the storage sheath 30 and does not receive a force from the central shaft 33. This form of the expansion body 21 can be defined as being included in the reference form.

Next, the operator operates the operation unit 23 in a state where the atrial septum HA is held by the holding portion 51, to move the central shaft 33 to the proximal side. Accordingly, as illustrated in Fig. 6, the expansion body 21 receiving a compression force in the axial direction has an expanded form where the expansion body 21 is more expanded in the radial direction than in the reference form. In the expanded form of the expansion body 21, the proximal side holding portion 52 and the distal side holding portion 53 approach each other and the atrial septum HA is held between the proximal side holding portion 52 and the distal side holding portion 53. The holding portion 51 additionally expands in a state where the holding portion 51 holds the atrial septum HA, to widen the held through-hole Hh in the radial direction.

After the through-hole Hh is expanded, hemodynamics is confirmed. As illustrated in Fig. 4, the operator delivers a hemodynamics confirmation device 100 to the right atrium HRa via the inferior vena cava Iv. For example, a known echo catheter can be used as the hemodynamics confirmation device 100. The operator can cause a display device such as a display to display an echo image acquired by the hemodynamics confirmation device 100, and confirm the amount of blood passing through the through-hole Hh, based on a display result.

Next, the operator performs a maintenance treatment to maintain the size of the through-hole Hh. In the maintenance treatment, energy is applied to an edge portion of the through-hole Hh through the energy transmission element 22, so that the edge portion of the through-hole Hh is cauterized (heated and cauterized) by the energy. When a biological tissue in the vicinity of the edge portion of the through-hole Hh is cauterized through the energy transmission element 22, a degenerated portion in which the biological tissue is degenerated is formed in the vicinity of the edge portion. Since the biological tissue in the degenerated portion is in a state where elasticity is lost, the through-hole Hh can maintain a shape when the through-hole Hh is widened by the expansion body 21.

In addition, the energy transmission element 22 is disposed on the projection portion 54 of the proximal side holding portion 52. For this reason, the maintenance treatment is performed in a state where the energy transmission element 22 is buried in the biological tissue by pressing the projection portion 54 against the atrial septum HA. Accordingly, the energy transmission element 22 does not come into contact with the blood during the maintenance treatment, so that it is possible to suppress the generation of blood clots or the like caused by the leakage of a current to the blood.

After the maintenance treatment, hemodynamics is confirmed again, and when the amount of the blood passing through the through-hole Hh reaches a desired amount, the operator reduces the expansion body 21 in diameter. The operator moves the storage sheath 30 with respect to the expansion body 21 in a distal end direction. Accordingly, the expansion body 21 is gradually stored in the storage sheath 30 from the proximal side. As illustrated in Figs. 7(A), 8, and 9, when the proximal side outward projection portion 55 of the expansion body 21 is stored in the storage sheath 30, the proximal side outward projection portion 55 is deformed into a shape close to being flat. At this time, the contact portion 58 of the inward projection portion 56 is pressed against the outer peripheral surface of the central shaft 33 by a reaction of the proximal side outward projection portion 55 that is elastically deformed into a shape close to being flat. Then, the contact portion 58 of the inward projection portion 56 slides on the outer peripheral surface of the central shaft 33 in a contact state, and moves to the distal side with respect to the central shaft 33. At this time, the outer peripheral surface of the central shaft 33 having a cylindrical shape is curved to project outward in the radial direction, so that the outer peripheral surface forms a protruding portion 34. For this reason, the protruding portion 34 that is a part of the outer peripheral surface of the central shaft 33 enters a space surrounded by the opening portion 59 of the contact portion 58. The protruding portion 34 in the outer peripheral surface of the central shaft 33 extends in the axial direction. For this reason, as indicated by an alternate long and short dash line in Fig. 8, the displacement of the inward projection portion 56 including the contact portion 58 from the outer peripheral surface of the central shaft 33 is suppressed. In a case where the inward projection portion 56 is displaced from the outer peripheral surface of the central shaft 33, when the expansion body 21 is stored in the storage sheath 30, the expansion body 21 may be damaged due to receiving an excessive load or may be difficult to store in the storage sheath 30. However, in the present embodiment, since the inward projection portion 56 is unlikely to be displaced from the outer peripheral surface of the central shaft 33, it is possible to suppress damage to the expansion body 21 or to suppress the difficulty in storing the expansion body 21 in the storage sheath 30 when stored in the storage sheath 30.

The operator additionally moves the storage sheath 30 to the distal side with respect to the expansion body 21. Accordingly, as illustrated in Fig. 7(B), the inward projection portion 56 and the distal side outward projection portion 57 of the expansion body 21 are gradually stored in the storage sheath 30 from the proximal side. When the distal side outward projection portion 57 of the expansion body 21 is stored in the storage sheath 30, the distal side outward projection portion 57 is deformed into a shape close to being flat. At this time, the contact portion 58 of the inward projection portion 56 is pressed against the outer peripheral surface of the central shaft 33 by a reaction of the distal side outward projection portion 57 that is elastically deformed into a shape close to being flat. However, since the protruding portion 34 of the central shaft 33 enters the space surrounded by the opening portion 59 of the contact portion 58, the displacement of the inward projection portion 56 including the contact portion 58 from the outer peripheral surface of the central shaft 33 is suppressed. For this reason, it is possible to suppress damage to the expansion body 21 or to suppress the difficulty in storing the expansion body 21 in the storage sheath 30 when stored in the storage sheath 30.

Note that the configuration where the contact portion 58 of the expansion body 21 stored in the storage sheath 30 includes the opening portion 59 that the protruding portion 34 of the outer peripheral surface of the central shaft 33 enters is also effective when the expansion body 21 is released from the storage sheath 30 and is expanded. Namely, the inward projection portion 56 including the contact portion 58 is unlikely to be displaced from the outer peripheral surface of the central shaft 33, so that the expansion body 21 can be smoothly expanded.

After the operator stores the expansion body 21 in the storage sheath 30, the operator additionally removes the entirety of the medical device 10 out of the living body to end the treatment.

As described above, the medical device 10 according to the aforementioned embodiment includes the shaft portion 20 that is elongate; and the expansion body 21 provided at the distal portion of the shaft portion 20 to be expandable and contractable in a radial direction. The shaft portion 20 includes the central shaft 33 extending along an axis of the expansion body 21. The expansion body 21 includes the plurality of wire portions 50 that are expandable and contractable in the radial direction. Each of the wire portions 50 includes the contact portion 58 that comes into contact with the central shaft 33, when contracted. The opening portion 59 is formed in the contact portion 58. The protruding portion 34 is formed in the central shaft 33. When the expansion body 21 is contracted, at least a part of the protruding portion 34 can enter an inside of the opening portion 59, and come into contact with the opening portion 59. For this reason, when the expansion body 21 is stored in the storage sheath 30, the medical device 10 can suppress the displacement of the contact portion 58 of the expansion body 21 to contract, from the central shaft 33. For this reason, it is possible to suppress damage to the expansion body 21 or to suppress the difficulty in storing the expansion body 21 in the storage sheath 30 when the expansion body 21 is stored in the storage sheath 30.

In addition, the opening portion 59 and/or the protruding portion 34 extend in the axial direction. Accordingly, even when the axial positions of the contact portion 58 of the expansion body 21 to contract and of the central shaft 33 are changed, it is possible to suppress the displacement of the contact portion 58 from the central shaft 33.
Note that in a case where one of the opening portion 59 and the protruding portion 34 extends in the axial direction, even when the other does not extend in the axial direction, the opening portion 59 and the protruding portion 34 are slidable on each other without being displaced from each other. Note that both the opening portion 59 and the protruding portion 34 may extend in the axial direction.

In addition, when the expansion body 21 is contracted, the contact portion 58 or the central shaft 33 comes into contact with the opening portion 59 so as to be slidable in the axial direction. Accordingly, in the medical device 10, when the expansion body 21 is stored in the storage sheath 30, the contact portion 58 of the expansion body 21 to contract is slidable in the axial direction without being displaced from the central shaft 33. For this reason, it is possible to suppress damage to the expansion body 21 or to suppress the difficulty in storing the expansion body 21 in the storage sheath 30 when the expansion body 21 is stored in the storage sheath 30.

In addition, the wire portion 50 includes the proximal side outward projection portion 55 protruding outward in the radial direction, the distal side outward projection portion 57 located closer to the distal side than the proximal side outward projection portion 55, to protrude outward in the radial direction, and the inward projection portion 56 protruding inward in the radial direction between the proximal side outward projection portion 55 and the distal side outward projection portion 57. The contact portion 58 is formed in the inward projection portion 56. Accordingly, when the proximal side outward projection portion 55 is contracted and stored in the storage sheath 30 from the proximal side, the contact portion 58 of the inward projection portion 56 can come into contact with the central shaft 33. At this time, since the protruding portion 34 can enter and come into contact with the opening portion 59, when the proximal side outward projection portion 55 is stored in the storage sheath 30, it is possible to suppress the displacement of the contact portion 58 from the central shaft 33. In addition, also when the inward projection portion 56 and the distal side outward projection portion 57 are stored in the storage sheath 30, since the protruding portion 34 can enter and come into contact with the opening portion 59, it is possible to suppress the displacement of the contact portion 58 from the central shaft 33. For this reason, it is possible to suppress damage to the expansion body 21 or to suppress the difficulty in storing the expansion body 21 in the storage sheath 30 when the expansion body 21 including the proximal side outward projection portion 55, the inward projection portion 56, and the distal side outward projection portion 57 is stored in the storage sheath 30.

In addition, the medical device 10 includes the energy transmission element 22 that is disposed on the wire portion 50 to output energy. Accordingly, it is possible to suppress the displacement of the contact portion 58 of the wire portion 50 on which the energy transmission element 22 is disposed, from the central shaft 33.

In addition, the opening portion 59 is a through-hole. Accordingly, the structure of the medical device 10 can be simplified to reduce the diameter and to reduce the cost.

In addition, the axially orthogonal cross section of the outer peripheral surface of the central shaft 33 is a substantially circular shape, and the protruding portion 34 is a part of the outer peripheral surface of the central shaft 33. Accordingly, the structure of the medical device 10 can be simplified to reduce the diameter and to reduce the cost. In addition, since the protruding portion 34 is smooth due to the outer peripheral surface of the central shaft 33 serving as the protruding portion 34, it is possible to suppress the hooking of other instruments or of other portions of the medical device 10 on the protruding portion 34.

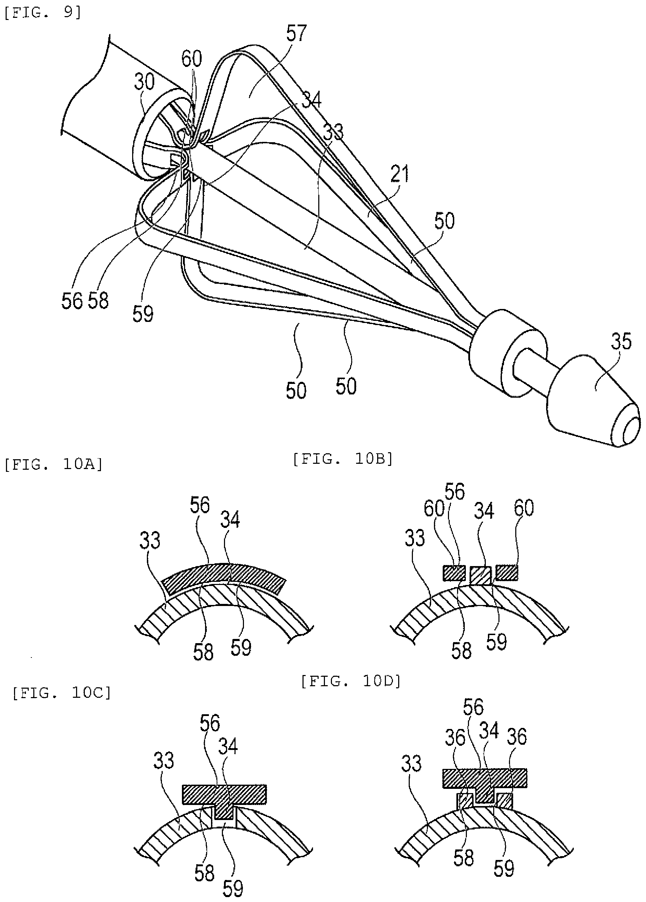

As in a first modification example illustrated in Fig. 10(A), the opening portion 59 may be a non-penetrating recessed portion.
Accordingly, the opening portion 59 is not penetrated to a side opposite a side on which the contact portion 58 of the inward projection portion 56 is provided. For this reason, it is possible to suppress interference of the opening portion 59 with other instruments or with other portions of the medical device 10.

In addition, as in a second modification example illustrated in Fig. 10(B), the protruding portion 34 may be a member that is fixed to the outer peripheral surface of the central shaft 33 to protrude outward in the radial direction from the outer peripheral surface. Accordingly, the protruding portion 34 protruding from the outer peripheral surface of the central shaft 33 can reliably enter the inside of the opening portion 59 of the contact portion 58, and come into contact with the opening portion 59. For this reason, the protruding portion 34 can effectively suppress the displacement of the protruding portion 34 that has entered the inside of the opening portion 59, from the opening portion 59.

In addition, as in a third modification example illustrated in Fig. 10(C), the central shaft 33 may be provided with the opening portion 59, and the inward projection portion 56 of the expansion body 21 may be provided with the protruding portion 34. In addition, the central shaft 33 may be provided with both an opening portion and a protruding portion, and the inward projection portion 56 may be provided with a protruding portion that comes into contact with the opening portion of the central shaft 33, and with an opening portion that comes into contact with the protruding portion of the central shaft 33.

In addition, as in a fourth modification example illustrated in Fig. 10(D), the opening portion 59 may be formed of two opening side protruding portions 36 that are arranged at an interval on the outer peripheral surface of the central shaft 33. Accordingly, the opening side protruding portions 36 can effectively suppress the displacement of the protruding portion 34 of the inward projection portion 56 that has entered the inside of the opening portion 59, from the opening portion 59.

In addition, the central shaft 33 which faces the contact portion 58 and in which the opening portion 59 or the protruding portion 34 is formed may not be a shaft for pulling to cause a compression force to act on the expansion body 21.

### Reference Signs List

- 10:: Medical device
- 20:: Shaft portion
- 21:: Expansion body
- 22:: Energy transmission element
- 30:: Storage sheath
- 33:: Central shaft
- 36:: Opening side protruding portion
- 34:: Protruding portion
- 50:: Wire portion
- 55:: Proximal side outward projection portion
- 56:: Inward projection portion
- 57:: Distal side outward projection portion
- 58:: Contact portion
- 59:: Opening portion

## Claims

1. A medical device (10) comprising:
a shaft portion (20) that is elongate; and
an expansion body (21) provided at a distal portion of the shaft portion (20) to be expandable and contractable in a radial direction,
wherein the shaft portion (20) includes a central shaft (33) extending along an axis of the expansion body (21),
the expansion body (21) includes a plurality of wire portions (50) that are expandable and contractable in the radial direction,
each of the wire portions (50) includes a contact portion (58) that comes into contact with the central shaft (33), when contracted, **characterised in that**
an opening portion (59) is formed in at least one of the contact portion (58) and the central shaft (33),
a protruding portion (34) is formed in at least the other one of the contact portion (58) and the central shaft (33), and
when the expansion body (21) is contracted, at least a part of the protruding portion (34) enters an inside of the opening portion (59), and comes into contact with the opening portion (59).

2. The medical device (10) according to claim 1,
wherein the opening portion (59) and/or the protruding portion (34) extend in an axial direction.

3. The medical device (10) according to claim 1 or 2,
wherein when the expansion body (21) is contracted, the contact portion (58) or the central shaft (33) comes into contact with the opening portion (59) so as to be slidable in an axial direction.

4. The medical device (10) according to any one of claims 1 to 3,
wherein the wire portion (50) includes a proximal side outward projection portion (55) protruding outward in the radial direction, a distal side outward projection portion (57) located closer to a distal side than the proximal side outward projection portion (55), to protrude outward in the radial direction, and an inward projection portion (56) protruding inward in the radial direction between the proximal side outward projection portion (55) and the distal side outward projection portion (57), and
the contact portion (58) is formed in the inward projection portion (56).

5. The medical device (10) according to any one of claims 1 to 4, further comprising:
an energy transmission element (22) disposed on the wire portion (50) to output energy.

6. The medical device (10) according to any one of claims 1 to 5,
wherein the opening portion (59) is a through-hole.

7. The medical device (10) according to any one of claims 1 to 5,
wherein the opening portion (59) is a non-through recessed portion.

8. The medical device (10) according to any one of claims 1 to 7,
wherein an axially orthogonal cross section of an outer peripheral surface of the central shaft (33) is a substantially circular shape, and
the protruding portion (34) is a part of the outer peripheral surface of the central shaft (33).

9. The medical device (10) according to any one of claims 1 to 7,
Wherein the protruding portion (34) is a member protruding outward in the radial direction from an outer peripheral surface of the central shaft (33).

10. The medical device (10) according to any one of claims 1 to 9,
wherein the opening portion (59) is formed of two opening side protruding portions (36) arranged at an interval.

## Patentansprüche

1. Medizinische Vorrichtung (10), umfassend:
einen Schaftabschnitt (20), der langgestreckt ist; und
einen Ausdehnungskörper (21), der in einem distalen Abschnitt des Schaftabschnitts (20) so vorgesehen ist, dass er in einer radialen Richtung ausdehnbar und zusammenziehbar ist,
wobei der Schaftabschnitt (20) einen zentralen Schaft (33) aufweist, der sich entlang einer Achse des Ausdehnungskörpers (21) erstreckt,
der Ausdehnungskörper (21) eine Vielzahl von Drahtabschnitten (50) umfasst, die in der radialen Richtung ausdehnbar und zusammenziehbar sind,
jeder der Drahtabschnitte (50) einen Kontaktabschnitt (58) umfasst, der mit dem zentralen Schaft (33) in Kontakt kommt, wenn er zusammengezogen ist, **dadurch gekennzeichnet, dass**
ein Öffnungsabschnitt (59) in mindestens einem von dem Kontaktabschnitt (58) und dem zentralen Schaft (33) ausgebildet ist,
ein vorstehender Abschnitt (34) in mindestens dem anderen von dem Kontaktabschnitt (58) und dem zentralen Schaft (33) ausgebildet ist, und
wenn der Ausdehnungskörper (21) zusammengezogen ist, mindestens ein Teil des vorstehenden Abschnitts (34) in das Innere des Öffnungsabschnitts (59) eintritt und mit dem Öffnungsabschnitt (59) in Kontakt kommt.

2. Medizinische Vorrichtung (10) nach Anspruch 1,
wobei sich der Öffnungsabschnitt (59) und/oder der vorspringende Abschnitt (34) in einer axialen Richtung erstrecken.

3. Medizinische Vorrichtung (10) nach Anspruch 1 oder 2,
wobei, wenn der Ausdehnungskörpers (21) zusammengezogen ist, der Kontaktabschnitt (58) oder der zentrale Schaft (33) mit dem Öffnungsabschnitt (59) in Kontakt kommen, sodass er in einer axialen Richtung verschiebbar ist.

4. Medizinische Vorrichtung (10) nach einem der Ansprüche 1 bis 3,
wobei der Drahtabschnitt (50) einen auf der proximalen Seite liegenden, nach außen gerichteten Vorsprungsabschnitt (55), der in der radialen Richtung nach außen ragt, einen auf der distalen Seite liegenden, nach außen gerichteten Vorsprungsabschnitt (57), der näher an einer distalen Seite als der auf der proximalen Seite liegende, nach außen gerichtete Vorsprungsabschnitt (55) angeordnet ist, um in der radialen Richtung nach außen zu ragen, und einen nach innen gerichteten Vorsprungsabschnitt (56) aufweist, der in der radialen Richtung zwischen dem auf der proximalen Seite liegenden, nach außen gerichteten Vorsprungsabschnitt (55) und dem auf der distalen Seite liegenden, nach außen gerichteten Vorsprungsabschnitt (57) nach innen ragt, und
der Kontaktabschnitt (58) in dem nach innen gerichteten Vorsprungsabschnitt (56) ausgebildet ist.

5. Medizinische Vorrichtung (10) nach einem der Ansprüche 1 bis 4, ferner umfassend:
ein Energieübertragungselement (22), das an dem Drahtabschnitt (50) angeordnet ist, um Energie abzugeben.

6. Medizinische Vorrichtung (10) nach einem der Ansprüche 1 bis 5,
wobei der Öffnungsabschnitt (59) ein Durchgangsloch ist.

7. Medizinische Vorrichtung (10) nach einem der Ansprüche 1 bis 5,
wobei der Öffnungsabschnitt (59) ein nicht durchgehender vertiefter Abschnitt ist.

8. Medizinische Vorrichtung (10) nach einem der Ansprüche 1 bis 7,
wobei ein axialer orthogonaler Querschnitt einer äußeren Umfangsfläche des zentralen Schaftes (33) eine im Wesentlichen kreisförmige Form aufweist, und
der vorstehende Abschnitt (34) ein Teil der äußeren Umfangsfläche des zentralen Schaftes (33) ist.

9. Medizinische Vorrichtung (10) nach einem der Ansprüche 1 bis 7,
wobei der vorstehende Abschnitt (34) ein Element ist, das in radialer Richtung von einer äußeren Umfangsfläche des zentralen Schaftes (33) nach außen vorsteht.

10. Medizinische Vorrichtung (10) nach einem der Ansprüche 1 bis 9,
wobei der Öffnungsabschnitt (59) aus zwei in einem Abstand angeordneten, auf der Öffnungsseite liegenden, vorspringenden Abschnitten (36) ausgebildet ist.

## Revendications

1. Dispositif médical (10) comprenant :
une partie de la tige (20) qui est allongée ; et
un corps d'expansion (21) disposé sur une partie distale de la partie de la tige (20) pour être extensible et contractable dans une direction radiale,
dans lequel la partie de l'arbre (20) comprend un arbre central (33) s'étendant le long d'un axe du corps d'expansion (21),
le corps d'expansion (21) comprend une pluralité de portions de fil (50) qui sont expansibles et contractables dans la direction radiale,
chacune des portions de fil (50) comprend une portion de contact (58) qui entre en contact avec l'arbre central (33) lorsqu'elle est contractée, **caractérisée en ce qu'**une portion d'ouverture (59) est formée dans au moins l'une de la portion de contact (58) et l'arbre central (33),
une partie faisant saillie (34) est formée dans au moins l'autre partie de contact (58) et l'arbre central (33), et
lorsque le corps d'expansion (21) est contracté, au moins une partie de la partie faisant saillie (34) pénètre à l'intérieur de la partie d'ouverture (59) et entre en contact avec la partie d'ouverture (59).

2. Dispositif médical (10) selon la revendication 1, dans lequel la portion d'ouverture (59) et/ou la portion faisant saillie (34) s'étendent dans une direction axiale.

3. Dispositif médical (10) selon la revendication 1 ou la revendication 2, dans lequel, lorsque le corps d'expansion (21) est contracté, la partie de contact (58) ou l'arbre central (33) entre en contact avec la partie d'ouverture (59) de manière à pouvoir coulisser dans une direction axiale.

4. Dispositif médical (10) selon l'une quelconque des revendications 1 à 3,
dans lequel la portion de fil (50) comprend une portion de projection vers l'extérieur du côté proximal (55) faisant saillie vers l'extérieur dans la direction radiale, une portion de projection vers l'extérieur du côté distal (57) située plus près d'un côté distal que la portion de projection vers l'extérieur du côté proximal (55), pour faire saillie vers l'extérieur dans la direction radiale, et une portion de projection vers l'intérieur (56) faisant saillie vers l'intérieur dans la direction radiale entre la portion de projection vers l'extérieur du côté proximal (55) et la portion de projection vers l'extérieur du côté distal (57), et
la partie de contact (58) est formée dans la partie de projection vers l'intérieur (56).

5. Dispositif médical (10) selon l'une quelconque des revendications 1 à 4, comprenant en outre :
un élément de transmission d'énergie (22) disposé sur la partie filaire (50) pour produire de l'énergie.

6. Dispositif médical (10) selon l'une quelconque des revendications 1 à 5, dans lequel la partie d'ouverture (59) est un trou traversant.

7. Dispositif médical (10) selon l'une quelconque des revendications 1 à 5, dans lequel la partie d'ouverture (59) est une partie en retrait non traversante.

8. Dispositif médical (10) selon l'une quelconque des revendications 1 à 7, dans lequel une section transversale axialement orthogonale d'une surface périphérique extérieure de l'arbre central (33) est de forme sensiblement circulaire, et
la partie faisant saillie (34) fait partie de la surface périphérique extérieure de l'arbre central (33).

9. Dispositif médical (10) selon l'une quelconque des revendications 1 à 7, dans lequel la partie faisant saillie (34) est un élément faisant saillie vers l'extérieur dans la direction radiale à partir d'une surface périphérique extérieure de l'arbre central (33) .

10. Dispositif médical (10) selon l'une quelconque des revendications 1 à 9, dans lequel la partie d'ouverture (59) est formée de deux parties faisant saillie du côté de l'ouverture (36) disposées à intervalle.
